# FASCICULE DE BREVET EUROPEEN

(11) **EP 0 728 307 B1**
(45) Date de publication et mention de la délivrance du brevet: **26.03.2003**
(21) Numéro de dépôt: 95929926.4
(22) Date de dépôt: 04.09.1995
(51) Int. Cl.: G01N 33/28, G01N 33/24, G01N 25/00, G01N 7/00

(54) **DISPOSITIF POUR FAIRE DES MESURES THERMODYNAMIQUES SUR DES FLUIDES POLYPHASIQUES A TRES HAUTES PRESSIONS ET TEMPERATURES**
VORRICHTUNG ZUM AUSFÜHREN THERMODYNAMISCHER MESSUNGEN AN SICH UNTER SEHR HOHEN DRÜCKEN UND TEMPERATUREN BEFINDLICHEN POLYPHASISCHEN FLÜSSIGKEITEN
DEVICE FOR PERFORMING THERMODYNAMIC MEASUREMENTS ON MULTIPHASE FLUIDS AT VERY HIGH PRESSURES AND TEMPERATURES

(30) Priorité: 09.09.1994 FR 9410784
(43) Date de publication de la demande: 28.08.1996
(73) Titulaire: INSTITUT FRANCAIS DU PETROLE, 92500 Rueil Malmaison (FR)
(72) Inventeur: MORACCHINI, Gérard Les Hauts-de-Maugarny, F-95580 Andilly (FR); SANCHEZ, José, F-95270 Viarmes (FR); DE HEMPTINNE, Jean-Charles, F-78110 Le Vésinet (FR); UNGERER, Philippe, F-94000 Créteil (FR)
(86) Numéro de dépôt international: FR9501146
(87) Numéro de publication internationale: WO96007902

(56) Documents cités:
- EP-A- 0 385 035
- EP-A- 0 473 472
- FR-A- 2 593 921
- JOURNAL OF PHYSICAL CHEMISTRY, vol. 85, no. 22, 1981 US, pages 3303-3307, A.Z. FRANCESCONI, ET AL. 'Phase Equilibria and PVT Data for the Methane-Methanol System to 300 MPa and 240 C'
- PATENT ABSTRACTS OF JAPAN vol. 18 no. 162 (P-1712) ,17 Mars 1994 & JP,A,05 332942 (JAPAN STEEL WORKS LTD) 17 Décembre 1993,

## Description

La présente invention concerne un dispositif pour mesurer des propriétés thermodynamiques d'échantillons de fluides à très haute pression et à température élevée, notamment de fluides corrosifs.

Le dispositif selon l'invention convient pour de nombreuses applications. On peut l'utiliser notamment dans le cadre d'études de propriétés thermodynamiques d'échantillons prélevés à l'origine dans des puits forés jusque dans des gisements souterrains, et placés en surface dans des conditions de pression et de température qui reproduisent les conditions régnant aux lieux de prélèvement. Ces échantillons contiennent notamment des hydrocarbures, des saumures, du dioxyde de carbone, de l'hydrogène sulfuré H2S etc.

On peut également l'utiliser pour des applications en génie chimique, pour l'étude de solutions de polymères par exemple.

Les mesures ont pour objet notamment d'évaluer la teneur des échantillons en effluents pétroliers. Les propriétés thermodynamiques sont calculées en utilisant des modèles compositionnels dans lesquels on intègre des données obtenues par l'analyse des échantillons. En faisant varier certains paramètres tels que la pression et la température par exemple, depuis des valeurs reproduisant celles qui existent à leur profondeur d'enfouissement jusqu'à celles régnant en surface. Par des méthodes d'analyse de ce type, on arrive à mesurer des propriétés importantes telles que les pressions de saturation.

Les échantillons sont généralement isolés dans des dispositifs comportant une enceinte thermostatée ou étuve, une ou plusieurs cellules de confinement, adaptés à les maintenir dans des conditions de pression analogues à celles qui règnent dans le sous-sol par exemple. La conduite de certaines opérations de mesure nécessite que l'on puisse prélever une fraction plus ou moins importante des échantillons contenus dans les cellules de confinement à l'intérieur de l'enceinte. Pour la facilité des manipulations et la rigueur des mesures, il est important de respecter certaines conditions:
- il faut que les prélèvements soient effectués de préférence dans les conditions de pression et de température régnant dans l'enceinte thermostatée et donc que la cellule de prélèvement puisse être couplée simplement à la cellule contenant la substance à prélever, et ensuite qu'elle puisse être dégagée de la même manière;
- il faut que le volume prélevé soit connu avec précision pour pouvoir mesurer facilement sa masse volumique et qu'il soit transférable pratiquement en totalité vers d'autres appareils de mesure;
- il faut encore que les manoeuvres de soutirage de la fraction prélevée et ensuite de transfert vers un appareil de mesure tel qu'un gazomètre, soient conduites de façon automatique pour maintenir facilement des valeurs de consigne.

Par le brevet français du demandeur FR 2.666.415, on connaît un dispositif destiné à conduire des opérations de mesure sur des échantillons. Il comporte principalement une enceinte climatique dans laquelle est placé un corps comportant deux chambres cylindriques de volume variable délimitées respectivement par deux pistons mobiles. L'une des chambres est pourvue d'une paroi transparente afin de faciliter le transfert complet d'une phase de l'échantillon vers l'autre chambre. Des moyens d'entraînement sont associés aux deux pistons pour les déplacer de façon indépendante ou coordonnée. Le dispositif fonctionne sous la conduite d'un microcalculateur.

Par le brevet FR-A- 2 706 612 (US 5 536 474) du demandeur également, on connaît également un dispositif pour transférer des échantillons sous pression comportant une cellule de prélèvement permettant de faciliter les manoeuvres de transfert d'échantillons vers des appareils de mesure extérieurs à une enceinte climatique.

Par le document "Journal of Physical Chemistry, vol.85, N°22, 1981, pp. 3303-3307, A.Z. Francesconi et al.", "Equilibria and PVT Data for the Methane-Methanol System to 300 MPa.", on connaît une cellule qui comporte un corps rigide de confinement pourvu d'une chambre de confinement allongée et de volume variable pour des échantillons fluides, délimitée par un piston mobile suivant la direction d'allongement de la chambre, un conduit de communication contrôlée avec l'extérieur de la chambre, un bloc transparent constituant la paroi terminale de la chambre de confinement, opposée au piston mobile, et des moyens de visualisation de l'intérieur de la chambre au travers du bloc transparent.

Le dispositif selon l'invention offre de grandes facilités à un opérateur pour faire des opérations de mesure très précises sur des échantillons de fluide extraits du sous-sol, éventuellement corrosifs, tenus à des pressions élevées pouvant atteindre 130 à 150 MPa, et à des températures élevées pouvant atteindre 250°C et même plus.

Le dispositif comporte une enceinte thermostatée, un corps rigide de confinement disposé dans cette enceinte, pourvu d'au moins une chambre ou cellule de confinement allongée et de volume variable pour des échantillons fluides, délimitée par un piston mobile suivant la direction d'allongement de la chambre des moyens de communication contrôlée avec l'extérieur de la chambre, un bloc transparent constituant la paroi terminale de la chambre de confinement, opposée au piston mobile, des moyens de fixation étanches du bloc transparent au corps, et des moyens de visualisation de l'intérieur de la chambre au travers du bloc transparent.

Il est caractérisé en ce que les moyens de communication comportent des conduits contrôlés par des vannes, débouchant dans la chambre au voisinage du bloc transparent et latéralement décalés de part et d'autre de la direction d'allongement de la chambre, et en ce qu'il comporte des moyens pour faire pivoter sélectivement la cellule autour d'un axe perpendiculaire à la direction d'allongement de la chambre de confinement, de manière à déplacer l'orifice d'au moins un des conduits au sein d'une phase d'un échantillon fluide à plusieurs phases stratifiées.

Cette disposition est intéressante notamment si l'on étudie des échantillons de fluide comportant des phases stratifiées. Les conduits débouchant dans la chambre de confinement étant latéralement décalés, une inclinaison suffisante de la cellule par pivotement de l'axe a pour effet de les faire déboucher au sein de deux phases différentes et donc de permettre des prélèvements sélectifs. On peut noter aussi qu'une inclinaison latérale de la cellule, d'un côté ou de l'autre améliore la vision que l'observateur peut avoir de l'orifice des deux conduits. En outre le pivotement alterné de l'axe permet une parfaite homogénéisation des échantillons.

Les moyens de visualisation comportent par exemple un endoscope associé au bloc transparent, permettant à l'opérateur de voir les orifices des conduits débouchant dans la chambre de confinement.

Suivant un mode de réalisation préféré, le dispositif comporte un élément de mesure de la pression régnant dans la chambre, qui est disposé dans un évidement ménagé dans le piston mobile. Comme élément de mesure, on utilise avantageusement un capteur de pression à membrane affleurante rapporté sur le piston mobile, de manière à améliorer sa sensibilité.

Avec cet agencement, la pression dans la chambre reste mesurable jusqu'à l'enfoncement complet du piston mobile. On évite aussi les volumes morts, inévitables quand le moyen de mesure de pression est placé comme c'est généralement le cas, à l'extérieur de la chambre et relié à elle par un conduit particulier.

Le dispositif peut comporter des moyens optiques de mesure des déplacements du piston mobile, offrant une grande précision dans la mesure des volumes.

Suivant un autre mode de réalisation, le dispositif comporte dans le même corps rigide, une deuxième chambre de confinement délimitée par un deuxième piston mobile, cette deuxième chambre étant pourvue d'au moins un conduit contrôlé par une vanne, et un moyen de distribution pour faire communiquer les conduits associés aux deux chambres entre eux et avec l'extérieur du corps.

La deuxième chambre peut être identique à la première avec deux conduits écartés latéralement l'un de l'autre et un moyen de mesure dans le piston mobile, et aussi éventuellement un bloc transparent pour en délimiter le fond.

Suivant un mode de réalisation, les deux chambres de confinement peuvent être adjacentes.

L'agencement du dispositif selon l'invention, apparaîtra mieux à la lecture de la description ci-après de modes de réalisation décrits à titre d'exemples non limitatifs, en se référant aux dessins annexés où :
- la Fig.1 montre schématiquement un corps de confinement à chambre unique;
- la Fig.2 montre schématiquement un corps de confinement pourvu de deux chambres adjacentes; et
- la Fig.2A montre schématiquement une cellule de prélèvement d'échantillon telle que décrite dans la demande de brevet français précitée.

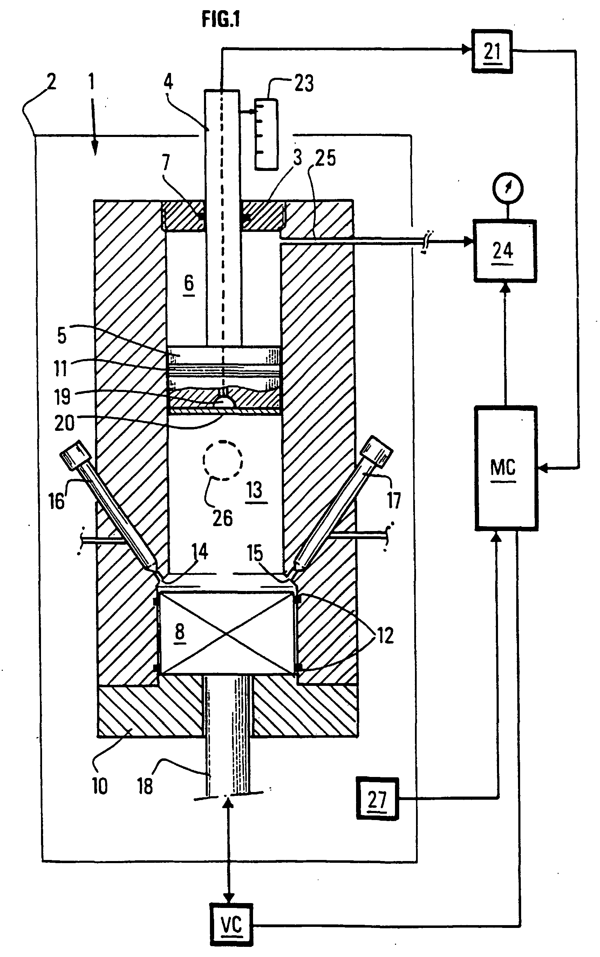

La cellule de confinement schématisée à la Fig.1, comporte un corps cylindrique allongé creux 1 que l'on place en opération dans une enceinte thermostatée 2 d'un type connu. A une première extrémité, le corps cylindrique 1 est fermé par un bouchon fileté 3 percé d'une ouverture de section adaptée à celle de la tige 4 d'un piston 5 déplaçable dans la cavité intérieure 6. Des joints d'étanchéité 7 d'un type connu tenant à des pressions élevées, sont disposés autour de la tige 4 pour isoler la cavité intérieure 6 de la chambre intérieure 13. A son extrémité opposée, le corps cylindrique 1 est fermé par un disque épais ou bloc 8 en matériau transparent tel que du saphir par exemple, qui est appliqué contre un épaulement intérieur 9 de la cavité intérieure 6 par un embout de fixation 10. Des joints d'étanchéité 11, 12 adaptés également aux très hautes pressions, sont disposés respectivement autour du piston 5 et du disque transparent 8 pour isoler la chambre intérieure 13 à volume variable délimitée par le corps cylindrique 1 entre le disque 8 et le piston mobile 5.

Au voisinage de la base de la cellule, du côté du disque transparent 8, débouchent deux conduits fins 14, 15 associés respectivement à deux vannes-pointeaux de contrôle 16, 17. Les orifices de ces deux conduits 14, 15 sont latéralement décalés l'un par rapport à l'autre et de préférence diamétralement opposés.

L'embout de fixation 10 comporte une ouverture pour le passage d'un moyen de visualisation 18 tel qu'un endoscope d'un type connu dont l'extrémité est appliquée contre le hublot 8. Une caméra vidéo VC est associée à l'endoscope 18. Celui-ci est monté pivotant par rapport au corps 1. En l'inclinant de part et d'autre de l'axe du cylindre, on peut ainsi mieux visualiser le volume intérieur et notamment les conduits de prélèvement 14, 15.

La pression régnant dans la chambre 13 est mesurée par un capteur de pression 19 rapporté sur le piston 5. Il est logé par exemple dans une cavité ménagée dans le piston 5 suivant son axe. Des conducteurs électriques 21, passant par un canal fin dans l'axe de la tige 4, relient le capteur 19 à son élément de mesure associé 22.

Les déplacements du piston 5, directement proportionnels au volume de la chambre 13, sont mesurés par un élément optique 23 de type règle optique par exemple.

Des moyens hydrauliques tels qu'une pompe à double effet 24 communiquant avec l'intérieur de la cavité 7, par un canal 25 qui débouche derrière le piston 5, sont utilisés pour déplacer celui-ci et ainsi faire varier le volume de la chambre 13.

La cellule est solidaire d'un axe 26 perpendiculaire à son axe d'allongement. Des moyens moteurs (non représentés) permettent de faire pivoter l'axe 26 et la cellule alternativement dans un sens et dans le sens opposé. L'amplitude angulaire du pivotement peut atteindre 300° par exemple.

Le pivotement alternatif de la cellule peut être utilisé à des fins de mise à l'équilibre de l'échantillon polyphasique à l'intérieur de la cellule.

Un pivotement d'amplitude contrôlée permet aussi de faciliter les observations :
a) Quand l'échantillon dans la chambre 13 est constitué d'un fluide polyphasique avec stratification des phases, une inclinaison suffisante de la cellule peut avoir pour effet de déplacer les orifices des conduits 14 et 15 respectivement au sein de deux des phases. On peut ainsi procéder à une extraction sélective de l'une ou l'autre par ouverture contrôlée des vannes 16 ou 17.
b) L'inclinaison de la cellule a pour effet utile également de décaler le niveau des interfaces en face de l'endoscope 18 ce qui améliore les possibilités de contrôle de l'observateur.

Le dispositif comporte également un capteur de température 27. L'ensemble des moyens de mesure 19, 21, 27, la caméra vidéo VC et les moyens moteurs hydrauliques 24 sont sous le contrôle d'un micro-calculateur MC.

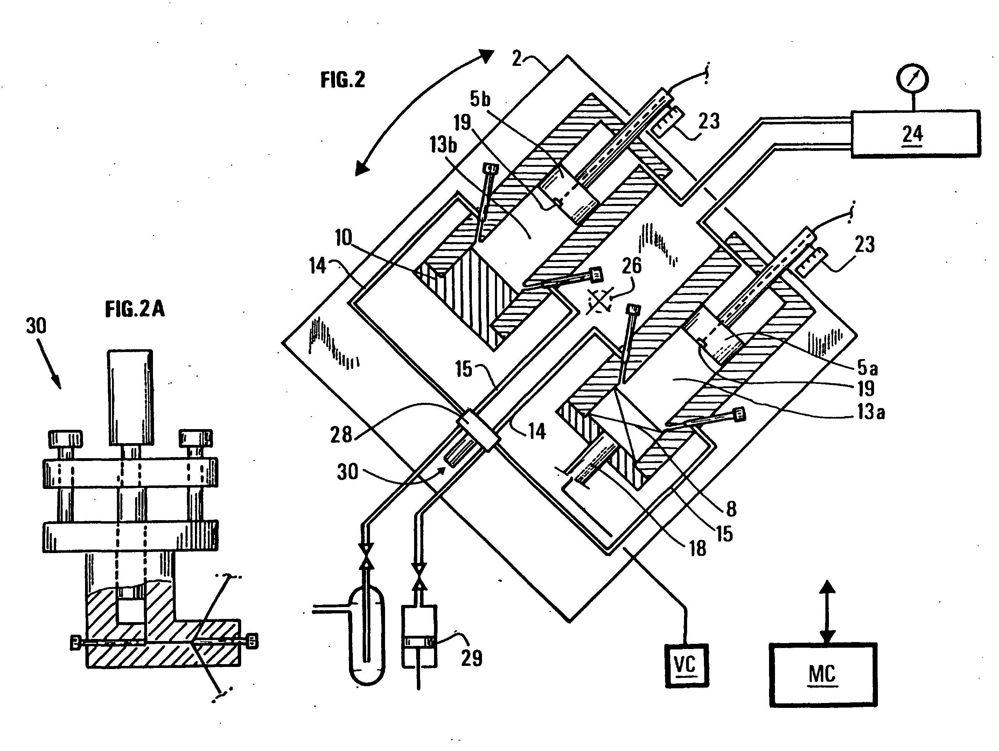

Suivant le mode de réalisation de la Fig.2, le dispositif comporte un corps rigide 1 à deux cellules de confinement d'axes parallèles disposées par exemple côte à côte. Les deux cellules comportent chacune une chambre (13A,13B) de volume variable délimitée par un piston (respectivement 5A, 5B) coulissant dans une cavité du corps et mu par les moyens hydrauliques 24. A chaque piston est associé un règle optique 23 de mesure de déplacement ainsi qu'un capteur de pression 19.

La chambre 13A est analogue à celle montrée à la Fig.1 avec son hublot épais 8, associé avec un moyen d'observation visuelle 18.

La deuxième cellule est dépourvue de moyen de contrôle visuels tels que 8, 18, le fond de la chambre 13B étant constitué d'un embout plein 10.

Les conduits 14, 15 issus des deux chambres 13A, 13B aboutissent à un élément de distribution 28 (manifold). En le manoeuvrant, on peut commander des transferts sélectifs de phases entre les deux chambres et/ou leur mise en communication avec des appareils extérieurs tels qu'une pompe d'injection de solvants 29. A cet élément de distribution 28, on peut également connecté une cellule de prélèvement 30 telle que celle décrite dans le brevet français précitée

Le corps 1 est pareillement mobile autour d'un axe pivotant 26 pour permettre une homogénéisation des échantillons ou faciliter leur contrôle. Les différents moyens de mesure dans les deux chambres 13A, 13B, les moyens moteurs actionnant les pistons SA, 5B et l'axe 26 et l'élément de distribution 28 sont sous le contrôle du micro-calculateur MC.

## Revendications

1. Dispositif pour mesurer des propriétés thermodynamiques d'un échantillon fluide à très haute pression et à température élevée, notamment de fluides corrosifs, comportant une enceinte thermostatée (2), un corps rigide de confinement (1) disposé dans cette enceinte, pourvu d'au moins une chambre ou cellule de confinement allongée et de volume variable (13) pour des échantillons fluides, délimitée par un piston (5) mobile suivant la direction d'allongement de la chambre des moyens de communication contrôlée avec l'extérieur de la chambre, un bloc transparent (8) constituant la paroi terminale de la chambre de confinement, opposée au piston mobile, des moyens (10) de fixation étanches du bloc transparent (8) au corps (1), et des moyens de visualisation (18, VC) de l'intérieur de la chambre au travers du bloc transparent, **caractérisé en ce que** les moyens de communication comportant des conduits (14, 15) contrôlés par des vannes (16, 17), débouchant dans la chambre au voisinage du bloc transparent et latéralement décalés de part et d'autre de la direction d'allongement de la chambre (13), et **en ce qu'**il comporte des moyens pour faire pivoter sélectivement la cellule autour d'un axe (26) perpendiculaire à la direction d'allongement de la chambre de confinement (13), de manière à déplacer l'orifice d'au moins un des conduits (14, 15) au sein d'une phase d'un échantillon fluide à plusieurs phases stratifiées.

2. Dispositif selon la revendication 1, **caractérisé en ce que** les moyens de visualisation comportent un endoscope (18) associé au bloc transparent (8).

3. Dispositif selon la revendication 2, **caractérisé en ce que** l'endoscope est monté pivotant pour permettre à un opérateur de visualiser les orifices des conduits (14, 15) débouchant dans la chambre de confinement (13).

4. Dispositif selon l'une des revendications précédentes, **caractérisé en ce qu'**il comporte un élément (19) de mesure de la pression régnant dans la chambre, qui est disposé dans un évidement ménagé dans le piston mobile (5).

5. Dispositif selon la revendication précédente, **caractérisé en ce que** l'élément de mesure (19) est un capteur de pression à membrane affleurante rapporté sur le piston mobile (5).

6. Dispositif selon l'une des revendications précédentes, **caractérisé en ce qu'**il comporte des moyens optiques (23) de mesure des déplacements du piston mobile (5).

7. Dispositif selon l'une des revendications précédentes, **caractérisé en ce que** le bloc transparent (8) est réalisé en saphir.

8. Dispositif selon l'une des revendications précédentes, **caractérisé en ce qu'**il comporte dans le même corps rigide, une deuxième chambre de confinement (13B) délimitée par un deuxième piston mobile (5B), cette deuxième chambre étant pourvue d'au moins un conduit contrôlé par une vanne, et un moyen de distribution (28) pour faire communiquer les conduits associés aux deux chambres entre eux et avec l'extérieur du corps.

9. Dispositif selon la revendication précédente, **caractérisé en ce que** les deux chambres de confinement (13A, 13B) sont disposées à côté l'une de l'autre.

## Claims

1. Device for measuring thermodynamic properties of a fluid sample at very high pressure and at high temperature, in particular of corrosive fluids, comprising a thermostat-controlled enclosure (2), a rigid confinement body (1) disposed in this enclosure, provided with at least one elongated confinement chamber or cell of variable volume (13) for fluid samples, delimited by a piston (5) mobile in the direction of elongation of the chamber, means for controlled communication with the exterior of the chamber, a transparent block (8) constituting the end wall of the confinement chamber, opposite the mobile piston, sealed means (10) for fixing the transparent block (8) to the body (1), and means (18, VC) for viewing the interior of the chamber through the transparent block, ***characterised in that*** the means of communication comprise passages (14, 15) controlled by valves (16, 17) debouching into the chamber in the vicinity of the transparent block and offset laterally on either side of the direction of elongation of the chamber (13), *and **in that*** it comprises means for selectively pivoting the cell about an axis (26) perpendicular to the direction of elongation of the confinement chamber (13) so as to move the orifice of at least one of the passages (14, 15) within a phase of a fluid sample with a plurality of stratified phases.

2. Device according to claim 1, ***characterised in that*** the means of viewing comprise an endoscope (18) associated with the transparent block (8).

3. Device according to claim 2, ***characterised in that*** the endoscope is mounted pivotably to allow an operator to view the orifices of the passages (14, 15) debouching into the confinement chamber (13).

4. Device according to one of the preceding claims, ***characterised in that*** it comprises an element (19) for measurement of the pressure prevailing in the chamber which is disposed in a recess formed in the mobile piston (5).

5. Device according to the preceding claim, ***characterised in that*** the measuring element (19) is a pressure pick-up with a flush diaphragm incorporated in the mobile piston (5).

6. Device according to one of the preceding claims, ***characterised in that*** it comprises optical means (23) for measurement of the movements of the mobile piston (5).

7. Device according to one of the preceding claims, ***characterised in that*** the transparent block (8) is made of sapphire.

8. Device according to one of the preceding claims, ***characterised in that*** it comprises in the same rigid body a second confinement chamber (13B) delimited by a second mobile piston (5B), this second chamber being provided with at least one passage controlled by a valve, and a means of distribution (28) to ensure communication of the passages associated with the two chambers with each other and with the exterior of the body.

9. Device according to the preceding claim, ***characterised in that*** the two confinement chambers (13A, 13B) are disposed beside one another.

## Patentansprüche

1. Verfahren zum Messen der thermodynamischen Eigenschaften einer Fluidprobe bei sehr hohem Druck und erhöhter Temperatur, insbesondere von korrosiven Fluiden, umfassend: eine thermostabilisierte Hülle (2), einen in dieser Hülle angeordneten steifen Begrenzungskörper (1), der mit wenigstens einer Kammer oder einer begrenzenden länglichen Zelle von variablem Volumen (13) für Fluidproben versehen ist, begrenzt durch einen Kolben (5), der gemäß der Längsrichtung der Kammer beweglich ist, Mittel zur geregelten Verbindung mit der Umgebung außerhalb der Kammer, einen transparenten die Endwandung der Begrenzungskammer bildenden Block (8), gegenüber dem beweglichen Kolben, dichte Befestigungsmittel (10) des transparenten Blocks (8) am Körper (1) und Mittel zur Sichtbarmachung (18, VC) des Inneren der Kammer durch den transparenten Block hindurch, **dadurch gekennzeichnet, dass** die Verbindungsmittel durch Ventile (16, 17) geregelte Leitungen (14, 15) umfassen, welche in der Kammer benachbart dem transparenten Block münden und zu beiden Seiten der Längsrichtung der Kammer (13) versetzt sind und dass sie Mittel umfasst, um selektiv die Zelle um eine Achse (26) senkrecht zur Längsrichtung der Begrenzungskammer (13) derart zu verschwenken, dass die Öffnung wenigstens einer der Leitungen (14, 15) inmitten einer Phase einer Fluidprobe mit mehreren geschichteten Phasen verschoben wird.

2. Vorrichtung nach Anspruch 1, **dadurch gekennzeichnet, dass** die Mittel zur Sichtbarmachung ein dem transparenten Block (8) zugeordnetes Endoskop (18) umfassen.

3. Vorrichtung nach Anspruch 2, **dadurch gekennzeichnet, dass** das Endoskop schwenkbar gelagert ist, um es einem Operateur zu ermöglichen, die Öffnungen der in die Begrenzungskammer (13) mündenden Leitungen (14, 15) sichtbar zu machen.

4. Vorrichtung nach einem der vorhergehenden Ansprüche, **dadurch gekennzeichnet, dass** sie ein Messelement (19) für den in der Kammer herrschenden Druck umfasst, welches in einer im beweglichen Kolben (5) vorgesehenen Ausnehmung angeordnet ist.

5. Vorrichtung nach dem vorhergehenden Anspruch, **dadurch gekennzeichnet, dass** das Messelement (19) ein Druckgeber mit versenkter Membran, angesetzt an den beweglichen Kolben (5), ist.

6. Vorrichtung nach einem der vorhergehenden Ansprüche, **dadurch gekennzeichnet, dass** sie optische Mittel (23) zum Messen der Verschiebungen des beweglichen Kolbens (5) umfasst.

7. Vorrichtung nach einem der vorhergehenden Ansprüche, **dadurch gekennzeichnet, dass** der transparente Block (8) aus Saphir gemacht ist.

8. Vorrichtung nach einem der vorhergehenden Ansprüche, **dadurch gekennzeichnet, dass** sie in dem gleichen steifen Körper eine zweite Begrenzungskammer (13B), die durch einen zweiten Kolben (5B) begrenzt ist, umfasst, wobei die zweite Kammer mit wenigstens einer durch ein Ventil geregelten Leitung versehen ist, und dass die Vorrichtung ein Verteilermittel (28) umfasst, um die den beiden Kammern zugeordneten Leitungen miteinander und mit der Umgebung außerhalb des Körpers in Verbindung zu setzen.

9. Vorrichtung nach dem vorhergehenden Anspruch, **dadurch gekennzeichnet, dass** die beiden Begrenzungskammem (13A, 13B) seitlich nebeneinander angeordnet sind.
